# EUROPEAN PATENT SPECIFICATION

(11) **EP 0 416 502 B1**
(45) Date of publication and mention of the grant of the patent: **30.03.1994**
(21) Application number: 90116873.2
(22) Date of filing: 03.09.1990
(51) Int. Cl.: A61K 35/78

(54) **A pharmaceutical composition having antitumor activity and preparation for its manufacture**
Pharmazeutische Zubereitung mit antitumoraler Wirkung und Verfahren zur Herstellung
Composition pharmaceutique ayant une activité antitumorale et préparation pour sa fabrication

(30) Priority: 04.09.1989 KR 8912750
(43) Date of publication of application: 13.03.1991
(73) Proprietor: Kim, Song Bae, Daejeon (KR)
(72) Inventor: Kim, Song Bae, Daejeon (KR)
(74) Representative: Kraus, Walter, Dr.

## Description

This invention relates to the use of Pulsatilla Radix and/or Clematis Radix for the manufacture of a pharmaceutical composition having antitumor activity.

It also relates to a novel pharmaceutical composition having antitumor activity containing Pulsatilla Radix(Pulsatilla Koreana Nakai, P. Cernua, P. davurica and P. ratinsis) and/or Clematis Radix; and one ingredient or more selected from bark of Ulmaceae species(Ulmus davidiana var. japonica); Armeniacae Semen;Panax Ginseng; and Glycyrrhizae Radix, and to a process for preparing the pharmaceutical composition.

The plants or Pulsatilla species are grown all over the world. The Pulsatilla Radix has been used as antiphlogistic agent, astringent, hemostatic and agent for dysentery in Korea. It is known that the Pulsatilla Radix contains anemonin, protoanemonin and saponin. Anemonin and protoanemonin have the following structures; 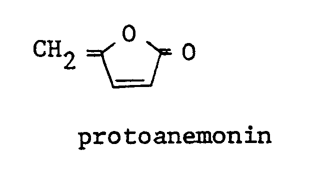 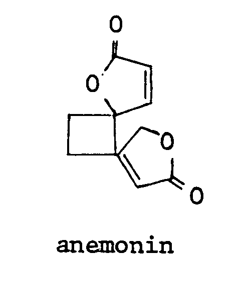
Protoanemonin is the precursor for the anemonin. The anemonin and protoanemonin are dissolved in water, alcohol, chloroform and chlorinated ethylene. Until now, it is not known that the Pulsatilla Radix has antitumor activity.

Clematis Radix(Root of Clematis mandshurica Maximowicz) contains anemonin, anemonol and saponin. It also has been used as agent for gout, diuretic and agent for difficult menstruum in Chinese medicine. But until now, it is not known that the Clematis Radix has antitumor activity. The bark of Ulmaceae species plant has mucin and tannin. Other ingredients than mucin and tannin are not known. It has been used as lenitive and binders in Chinese medicine. But it has not been used as anti-tumor agent.

Armeniacae Semen contains amygdalin, oil and emulsin and has been used as cough remedy, a base for ointment or solvent for injection. But until now it has not been used as antitumor agent. Panax Ginseng has been known from ancient times as marvellous medicine in the orient society. It has been used as tonic, agent for acute gastritis and agent for various bleeding diseases. In recent years, it is reported that Ginseng Radix has an anticarcinogenic effect. In Ginseng Radix, Ginseng saponin, essence oil, panaxtriol, beta-sisterol etc. are contained.

Glycyrrhizae Radix contains glycyrrhizin, liquiritin, licoricidin and liquiritoside and has been used as cough remedy, expectorant, diaphoretic and agent for gastritis. But, it is not known that the Glycyrrhizae Radix has anti-tumor activity.

The present inventor carried out an intensive study for natural substances and surprisingly found out that Pulsatilla Radix and/or Clematis Radix has excellent anti-tumor activity.

Accordingly, it is the object of this invention to provide the use of Pulsatilla Radix and/or Clematis Radix for the manufacture of a pharmaceutical composition having antitumor activity. It is also an object of the present invention to provide a pharmaceutical composition comprising Pulsatilla Radix and/or Clematis Radix in the form of a powder or extract and a powder or extract selected from Ulmaceae species, Armeniacae Semen, Panax Ginseng and Glycyrrhizae Radix, and a process for preparing such a composition.

This invention relates to the use of Pulsatilla Radix and/or Clematis Radix in the form of powder or extract extracted by a conventional solvent and of one or more ingredients selected from the group consisting carriers, antioxidants, preservatives, dissolving agents, hinders and solvents which are conventionally used in the pharmaceutical industry for the manufacture of a pharmaceutical composition having antitumor activity.

The pharmaceutical composition having antitumor activity may be in the form of tablet, capsule, injection, ointment, syrup, oral solution, oral suspension, or any other pharmaceutical preparation conventionally used in the pharmaceutical industry.

This invention relates also to a pharmaceutical composition having antitumor activity comprising Pulsatilla Radix and/or Clematis Radix in the form of powder or extract by a conventional solvent; and one or more ingredients selected from the group consisting bark of Ulmaceae species, Armeniacae Semen, Panax Ginseng and Glycyrrhizae Radix in the form of powder or extract extracted by a conventional solvent ; and one or more ingredients selected from carriers, antioxidants, preservatives, dissolving agents, binders and solvents which are conventionally used in the pharmaceutical industry.

The present inventor found out the fact that when one or more natural substances in the form of powder or extract selected from the group consisting bark of Ulmaceae, Armeniacae Semen, Panax Ginseng and Glycyrrhizae Radix are added to the Pulsatilla Radix and/or Clematis Radix in the form of powder or extract, the anti-tumor activity is much more strengthened.

This invention relates also to a process for the preparation of the above-mentioned pharmaceutical composition which comprises the steps of:
(a) powdering Pulsatilla Radix and/or Clematis Radix; or extracting Pulsatilla Radix and/or Clematis Radix by a conventional solvent used in pharmaceutical industry;
(b) powdering one or more ingredients selected from bark of Ulmaceae species, Armeniacae Semen, Panax Ginseng and Glycyrrhizae Radix; or extracting one or more ingredients selected from the group consisting bark of Ulmaceae species, Armeniacae Semen, Panax Ginseng and Glycyrrhizae Radix; and
(c) admixing the powdered or extracted Pulsatilla Radix and/or Clematis Radix; one or more ingredients selected from Ulmaceae species, Armeniacae Semen, Panax Ginseng and Glycyrrhizae Radix; and one or ingredients selected from carriers, antioxidants, preservatives, dissolving agents, binders and solvents which are conventionally used in the pharmaceutical industry.

The natural subtances used in the present invention may be 0-10 parts by weight of the Pulsatilla Radix, 0-10 parts by weight of the Clematis Radix(However, Pulsatilla Radix and Clematis Radix are not zero at the same time), 0-5 parts by weight of Panax Ginseng, 0-5 parts by weight of the bark of the Ulmaceae species, 0-3 parts by weight of the Armeniacae Semen and 0-5 parts by weight of the Glycyrrhizae Radix on the air-dried basis. They can be used in the form of powder or extract extracted by conventional solvent. One or more diluents selected from conventional carriers, antioxidants, preservatives, dissolving agents, disintegrators, lubricants binders and solvents may be added to the above ingredients. The natural substances of the present invention are air-dried and finely ground or extracted by water, lower alcohol, chloroform, methylenechloride or any other solvent which can extract active substances from the natural substances at the temperature of from 0°C to the boiling point of the solvent used for from 30 minutes to 24 hours. The solvent from the extract solution may be distilled off to obtain extract. The extrat may be dissolved in water, ethylalcohol or mixture thereof. When water is used as solvent, the solution may be directly used as pharmaceutical preparation without distillation of the water.

When each ingredients are used in the extracted form, each natural substances may be extracted separately or 2 kinds of the natural substances or more may be combined and extracted at the same time to obtain the extract. To the powdered or extracted natural substances, the following diluents may be added: carriers such as lactose, various starches, sucrose, mannitol, sorbitol, calcium sulfate, aluminium silicate, calcium sulfate, calcium carbonate; binders such as sucrose, glucose, starch paste, gelatin, carboxymethylcellulose, methylcellulose, gum arabic, gum tragacanth, ethylcellulose, sodium alginate, hydroxypropyl-methylcellulose, polyvinylpyrrolidone, soluble cellulose; disintegrators such as starch,carboxymethylcellulose, methylcellulose, crystalline cellulose; lubricanting agents such as magnesium stearate, calcium stearate; wetting agents such as glycerine, propylene glycol and sorbitol; preservatives such as sodium benzoate, methyl p-hydroxybenzoate, propyl p-hydroxybenzoate,benzalkonium chloride, chlorobutanol, sodium dehydroacetate, polymixin B sulfate; dissolving agents such as soluble alcohols and derivatives thereof, various surfactants; antioxidants such as sodium sulfite, sodium pyrosulfate, sodium metabisulfate, sodium bisulfite, rongalite, ascorbic acid; isotonic agents such as sodium chloride, dextrose; indolent agent such as benzylalcohol and chlorobutanol; and ointment base such as vaselline, fluid paraffin plastibase and various silicones, lard, various vegitable oils, waxes, refined lanolin.

About 0.5-10g of the Pulsatilla Radix and/or the Clematis Radix in the form of powder or extract (which is extracted from 0.5-10g of the Pulsatilla Radix and/or the Clematis Radix) may be administered for 1-10 times a day.

The present invention is explained in more detail with the following examples.

### Example 1

4g of air-dried Pulsatilla Radix, 2g of air-dried bark of Ulmus davidiana var. japonica and 1g of air-dried Glycyrrhizae Radix were finely ground, mixed uniformly and divided into each 1.5g of the mixture in vinylcoated envelope and sealed.

### Example 2

4g of air-dried Pulsatilla Radix, 2g of air-dried Clematis Radix, 2g of air-dried Ginseng Radix and 2g of Glycyrrhizae Radix were finely ground, mixed uniformly and divided into each 1.5g of the mixture in vinyl coated envelope and sealed.

### Example 3

6.26g of air-dried Pulsatilla Radix were added to 90ml of purified water and the mixture was warmed to 60°C and stirred for 60 minutes. The mixture was centrifuged at 3,500 RPM for about 30 minutes. About 60ml of the seperated solution was sterile-filtered in sterilization room at 60°C or below and the solution was made into isotonic solution by adding suitable amount of NaCl and the isotonic solution were sterile-filtered once again and divided to each 2.5ml of the solution in ampoule of 3ml at sterile state and sealed to obtain injection ampoule.

### Example 4

4g of air-dried and powdered Pulsatilla Radix, 2g of air-dried powdered bark of Ulmus davidiana var. japonica, 2g of air-dried and powdered Ginseng Radix and 1g of air-dried and powdered Glycyrrhizae Radix were added to 90ml of purified water and the mixture was stirred for 60 minutes at about 80°C by adding purified water corresponding to the water distilled off. The mixture was cooled to room temperature, centrifuged with 3,500 RPM for about 30 minutes to obtain about 46ml of solution. To the solution was added NaCl to obtain isotonic solution. The isotonic solution was filtered with conventional filtration in sterile room, sterile-filtered and divided into each 2ml of the solution in ampoule of 3ml, sealed and stored in refrigerator.

### Example 5

62.6g of air-dried and powdered Pulsatilla Radix, 31.3g of air-dried and powdered Ginseng Radix, 10g of air-dried and powdered Glycyrrhizae Radix were added to 900ml of purified water and the mixture was stirred for 60 minutes at about 60°C by adding purified water corresponding to the water distilled off. The mixture was filtered and the filtrate was concentrated to obtain about 26.4g of the extract.

### Example 6

6g of air-dried and powdered Clematis Radix, 3.13g of air-dried and powdered Ginseng Radix, 2g of Armemiacae Semen, and 1g of air-dried and powdered Glycyrrhizae Radix were added to 90ml of 40%(v/v) ethylalcohol and the mixture was stirred for about 120 minutes at about 40°C and extracted. The mixture was centrifuged with 3,500 RPM to obtain about 40ml of solution. The solution was concentrated to obtain about 2.50g of extract.

### Example 7

| | |
|---|---|
| Extract obtained in the Example 5 | 130mg |
| Sodium metabisulfite | 3.0mg |
| Methylparaben | 0.8mg |
| Propylparaben | 0.1mg |
| Isotonic solution, qs | to form 2ml |

The solution was filled into 2ml of ampoule by conventional method.

### Example 8

| | |
|---|---|
| Extract obtained in the Example 5 | 200mg |
| Crystalline cellulose | 50mg |
| Hydroxypropylcellulose | 17mg |
| Magnesium stearate | 3mg |

270mg of tablet was obtained by conventional method.

### Example 9

| | |
|---|---|
| Extract obtained in the Example 5 | 200mg |
| Talc | 10mg |
| Colloid silica | 5mg |
| Lactose | 85mg |

300mg of hard capsule was obtained by conventional method.

### Example 10

| | |
|---|---|
| Extract obtained in the Example 5 | 1000mg |
| Sucrose | 2000mg |
| Methylparaben | 20mg |
| Propylparaben | 5mg |
| Glycerine | 20mg |
| Sodium saccharin | 10mg |
| Orange essence, qs | |
| Purified water, qs | to form 100ml |

Oral solution was obtained by conventional method.

### Example 11

| | |
|---|---|
| Extract obtained in the Example 6 | 1.5g |
| Gum tragacanth | 1.0mg |
| Glycerine | 2ml |
| Purified water, qs | to form 50ml |

The above ingredients were stirred at room temperature and store for 24 hours to obtain linimenta.

### Experiment 1(Acute toxicity)

Animals: dd mice of body weight of 20-25g were used.
Method: The samples prepared in the Example 3 were administered through p.o., s.c., and i.v. respectively and the animals were watched for 72 hours whether the animals were died or not.

### Experiment 2(Acute toxicity)

Animals: Sprague-Dawley rats of body weight of 120-130g were used.
Method: The samples prepared in the Example 3 were administered through p.o., s.c., and i.v. respectively and the animals were watched for 72 hours whether the animals were died or not.
Results: The results of these experiments were shown in the Table I and II.

**Table I**

| Acute Toxicity of the present composition in mice | | | | | | | | |
|---|---|---|---|---|---|---|---|---|
| Route | Sample | Dose | No.of Animals | | MLD | | LD₅₀ | |
| | ml/20g | ml/kg | dosed | died | ml/20g | ml/kg | ml/20g | ml/kg |
| P.O. | 1.0 | 50 | 10 | 0 | >1.0 | >50 | - | |
| | 0.4 | 20 | 10 | 0 | | | | |
| S.C. | 0.8 | 40 | 10 | 0 | >0.8 | >40 | - | |
| | 0.4 | 20 | 10 | 0 | | | | |
| i.v. | 0.19 | 9.5 | 6 | 0 | | | | |
| | 0.22 | 11.0 | 6 | 1 | | | | |
| | 0.25 | 12.5 | 6 | 2 | - | | | |
| | 0.28 | 14.0 | 6 | 4 | | | | |
| | 0.30 | 15.0 | 6 | 6 | | | | |
| LD₅₀ value was calculated by Behren's method. | | | | | | | | |

**Table II**

| Acute Toxicity of Sample P in Rats | | | | | | | | |
|---|---|---|---|---|---|---|---|---|
| Route | Sample Dose | | No.of Animals | | MLD | | LD₅₀ | |
| | ml/20g | ml/kg | dosed | died | ml/20g | ml/kg | ml/200g | ml/kg |
| P.O. | 4.0 | 20 | 8 | 0 | 4.0 | 20 | - | |
| S.C. | 4.0 | 20 | 8 | 3 | 4.0 | 20 | - | |
| | 3.0 | 15 | 5 | 0 | | | | |

As shown in the table I and II, the pharmaceutical composition of the present invention has very week acute toxicity.

### Experiment 3(Antitumor effect against Sarcoma 180)

Animals: dd mice(male) of body weight of 20-25g were used.
Method: Sarcoma 180 tumor cells of 5X10⁶ were injected in the test mice. 7 days after, samples of 0.3ml of the present composition of the Example 3 were injected (s.c.) for 5 days. The animals were sacrificied after 30 days and the tumors were taken out and weighed. Additionally, the appearance tumor size was observed on the 10th day and 20th day respectively after the starting date of the administration of the present composition. 5-10 mice were used for each group.
Results: The results were shown in the Table III and IV.

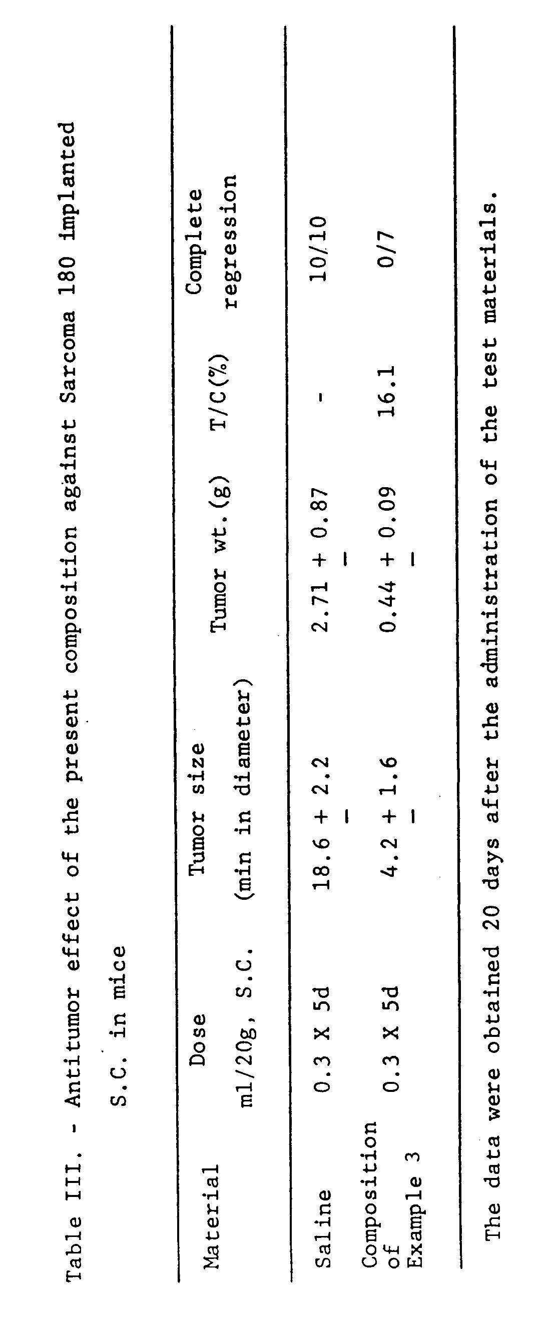

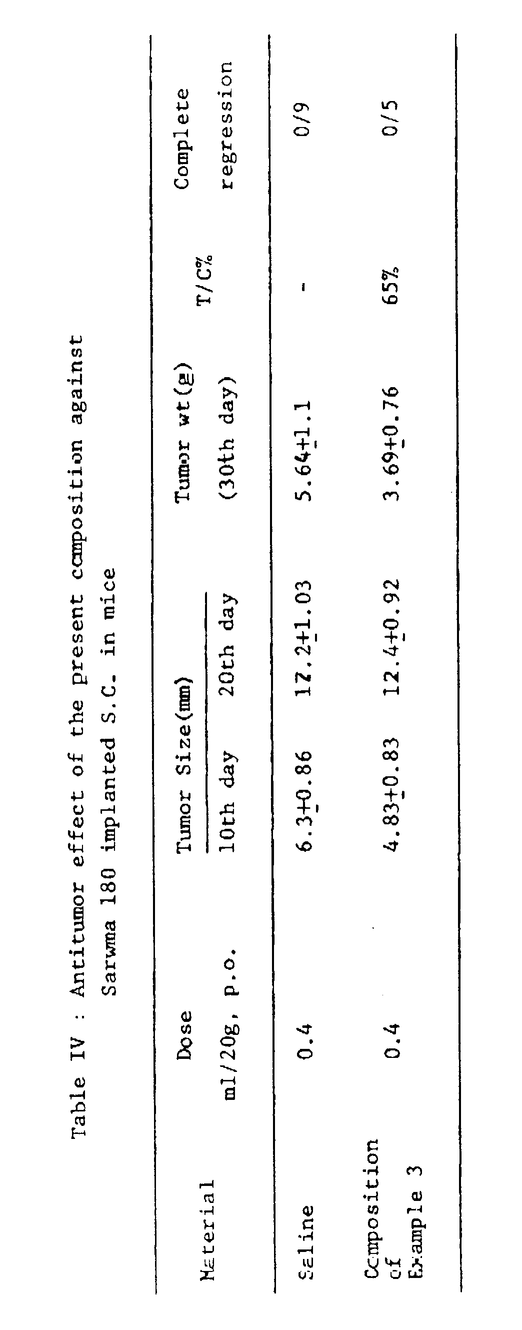

The mean tumor weight of the control group was 2.71g but the mean tumor weight of the group injected the present composition was 0.44g. According to the regulation of Cancer Institute of U.S.A., if the T/C(%) of any test compound is 42% or below, the compound is determined to be effective. As the T/C(%) of the present composition is 16.1%, the present composition is has an excellent effect against Sarcoma 180.

### Experiment 4(Clinical Test on volunteer)

Tested Person: Mr. Hyun Suck Suh aged 18 (Address: Majung-ri, Nam-myun, Buye-kun, Chungchungnam-do, Republic of Korea)
Kind of disease tumors of the lymph node on neck.
Diagnosis: At the Chungnam Medical College Hospital on July 17, 1973.
Period of medication: From September 20, 1973 to March 5, 1974.
Method of medication: The present composition prepared in the Example 3 was injected subcutaneously 2ml once a day for 1 month. Thereafter, the present composition was injected subcutaneously 4ml twice a day. After 2 months and 10 days from the start of medication, the tumors were disappeared. Thereafter, the medication was continuied for 3 months and 5 days.

After 3 years from the end, of medication, the Chungnam Medical College Hospital decided that the patient was completely cured.

### Experiment 5(Clinical Test on volunteer)

Tested person: Mr. Keun Bae Lee aged 66(Address: 793, Chungan-3-dong, Danbuk-myun, Euisung-kun, Kyungsangbuk-do, Republic of Korea).
Kind of Disease: Lung Cancer
Diagnosis: Kyemyung University attached Dong San Hospital on September 29, 1987.
Period of medication: From April 8, 1988 to November 23, 1988(for 7 months and 15 days).
Method of medication: The present composition prepared in the Example 7 was injected subcutaneously 2ml once a day. Kyemyung University attached Dong San Hospital reported that tumors were disappeared on December 5, 1988.
Figure 1 showed X-ray chart taken before the medication of the present composition;
Figure 2 showed X-ray chart taken 3 months after the medication of the present composition; and
Figure 3 showed X-ray chart taken after the end of the medication of the present composition.
As seen from the X-ray charts, the present composition has an excellent effect against lung cancer.

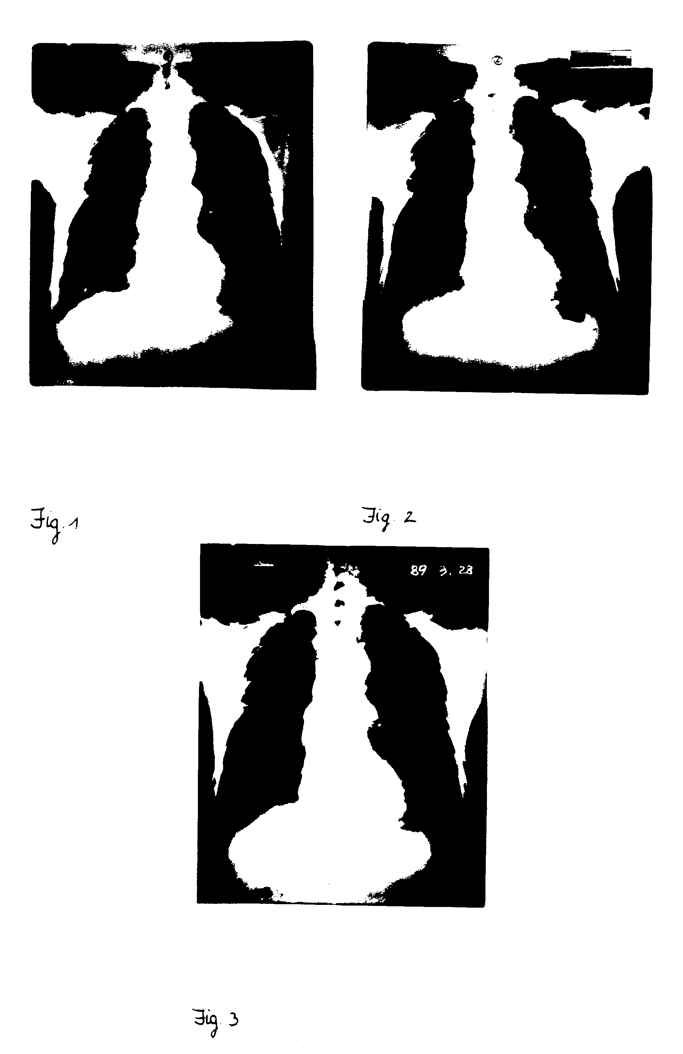

### Experiment 6(Clinical Test on volunteer)

Tested person: Mr. No charn Park aged 63 (Address: 196, Sukwan-ri, Nami-myun, Chungwon-kun, Chungchungbuk-do, Republic of Korea).
Kind of disease: Progressed stomach cancer.
Diagnosis: Chungnam University Medical College attached Hospital on March 15, 1988.
Period of medication: From April 8, 1988 to April 30, 1989(12 months and 22 days).
Method of medication: The present composition prepared in the Example 3 was injected(i.m.) 2ml once a day, injected(i.v.) 2ml once a day and administered orally 2.2ml once a day simultaneously. After one month of medication, about 20% of improvement effect was obtained. After 3 months of medication, about 30% of improvement effect was obtained. Thereafter, no improvement effect or ingravescence effect was appeared till 5th month of medication. Thereafter, the injections were maintained and the oral doses was doubled. About 80% of clinical effect of improvement was obtained at 6th month.
After 9th month of medication, all tumors were disappeared. From 10th month, only 2ml of the present composition once a day was injected(i.v.). Daejeon Chungang X-ray Clinic decided on March 25, 1989 that the patient was completely recovered.

### Experiment 7 (Clinical Test on volunteer)

Tested person: Mr. Duck Sang Kim Aged 46(Address: 90-20, Sajik-2-dong, Dongrae-ku, Pusan, Republic of Korea).
Kind of Disease: Progressed stomach cancer.
Diagnosis: Pusan Inje Medical College attached Baik Hospital on March 8, 1988.
Period of medication : From March 3, 1988 to February 3, 1989(10 months).
Method of medication : The present composition prepared in the Example 7 was injected(i.m.) 2ml once a day from April 3, 1988. A little amount of the present composition was orally administered simultaneously. From 20 days of medication, the injection method(i.m.) was changed into i.v. injection. After 3 months of medication, about 30% of improvement effect was obtained. After 5 months of medication, about 80% of improvement effect was obtained. From 6th month of medication, the amount of oral administration was doubled. After 8 months of medication, no tumors were appeared. Injection was ended from the 10th month of medication and only the oral administration was continued for one month. Pusan Inje Medical College attached Baik Hospital decided that the patient was completely recovered.

As seen from the said Experiments above, the present pharmaceutical composition has excellent antitumor activity.

## Claims

1. Use of Pulsatilla Radix and/or Clematis Radix in the form of powder or extract extracted by a conventional solvent and of one or more ingredients selected from carriers, antioxidants, preservatives, dissolving agents, binders and solvents which are conventionally used in the pharmaceutical industry for the manufacture of a pharmaceutical composition having antitumor activity.

2. A pharmaceutical composition having antitumor activity comprising Pulsatilla Radix and/or Clematis Radix in the form of powder or extract by a conventional solvent; and one or more ingredients selected from bark of Ulmaceae species, Armeniacae Semen, Panax Ginseng and Glycyrrhizae Radix in the form of powder or extract extracted by a conventional solvent ; and one or more ingredients selected from carriers, antioxidants, preservatives, dissolving agents, binders and solvents which are conventionally used in the pharmaceutical industry.

3. A process for the preparation of the pharmaceutical composition according to claim 2 which comprises the steps of;
(a) powdering Pulsatilla Radix and/or Clematis Radix; or extracting Pulsatilla Radix and/or Clematis Radix by a conventional solvent used in pharmaceutical industry;
(b) powdering one or more ingredients selected from bark of Ulmaceae species, Armeniacae Semen, Panax Ginseng and Glycyrrhizae Radix; or extracting one or more ingredients selected from the group consisting bark of Ulmaceae species, Armeniacae Semen, Panax Ginseng and Glycyrrhizae Radix; and
(c) admixing the powdered or extracted Pulsatilla Radix and/or Clematis Radix; one or more ingredients selected from Ulmaceae species, Armeniacae Semen, Panax Ginseng and Glycyrrhizae Radix; and one or ingredients selected from carriers, antioxidants, preservatives, dissolving agents, binders and solvents which are conventionally used in the pharmaceutical industry.

## Patentansprüche

1. Verwendung von Pulsatilla Radix und/oder Clematis Radix in Form eines Pulvers oder eines, mit einem üblichen Lösungsmittel extrahierten Extraktes und einem oder mehreren Bestandteilen, ausgewählt aus Trägern, Antioxidationsmitteln, Konservierungsmitteln, Auflösemitteln, Bindemitteln und Lösungsmitteln, die üblicherweise in der pharmazeutischen Industrie verwendet werden, für die Herstellung eines pharmazeutischen Präparats mit Antitumoraktivität.

2. Pharmazeutisches Präparat mit Antitumoraktivität, dadurch gekennzeichnet, daß es Pulsatilla Radix und/oder Clematis Radix in Form eines Pulvers oder eines Extrakts mit einem üblichen Lösungsmittel und einem oder mehreren Bestandteilen, ausgewählt aus der Rinde von Ulmaceae species, Armeniacae Semen, Panax Ginseng und Glycyrrhizae Radix, in Form eines Pulvers oder Extrakts, extrahiert mit einem üblichen Lösungsmittel und einen oder mehrere Bestandteile, ausgewählt aus der Gruppe Träger, Antioxidantien, Konservierungsmittel, Auflösemittel, Bindemittel und Lösungsmittel, die üblicherweise in der pharmazeutischen Industrie verwendet werden, enthält.

3. Verfahren zur Herstellung eines pharmazeutischen Präparats nach Anspruch 2, gekennzeichnet durch die Stufen:
(a) Pulverisierung von Pulsatilla Radix und/oder Clematis Radix oder Extraktion von Pulsatilla Radix und/oder Clematis Radix mit einem üblichen Lösungsmittel, das in der pharmazeutischen Industrie verwendet wird,
(b) Pulverisierung von einem oder mehreren Bestandteilen, ausgewählt aus der Rinde von Ulmaceae species, Armeniacae Semen, Panax Ginseng und Glycyrrhizae Radix, oder Extraktion von einem oder mehreren Bestandteilen, ausgewählt aus der Gruppe, die besteht aus der Rinde von Ulmaceae species, Armeniacae Semen, Panax Ginseng und Glycyrrhizae Radix, und
(c) Vermischen der gepulverten oder extrahierten Pulsatilla Radix und/oder Clematis Radix von einem oder mehreren Bestandteilen, ausgewählt aus Ulmaceae species, Armeniacae Semen, Panax Ginseng und Glycyrrhizae Radix, und einem oder mehreren Bestandteilen, ausgewählt aus Trägern, Antioxidantien, Konservierungsmitteln, Auflösemitteln, Bindemitteln und Lösungsmitteln, die üblicherweise in der pharmazeutischen Industrie verwendet werden.

## Revendications

1. Utilisation de Pulsatilla Radix et/ou Clematis Radix sous la forme d'une poudre ou d'un extrait obtenu au moyen d'un solvant usuel, et d'un ou plusieurs ingrédients choisis parmi des supports, des anti-oxydants, des agents de conservation, des agents de dissolution, des liants et des solvants, qu'on utilise habituellement dans l'industrie pharmaceutique pour la fabrication d'une composition pharmaceutique ayant une activité antitumorale.

2. Composition pharmaceutique ayant une activité antitumorale, comprenant Pulsatilla Radix et/ou Clematis Radix sous la forme d'une poudre ou d'un extrait obtenu au moyen d'un solvant usuel, et un ou plusieurs ingrédients choisis parmi l'écorce des espèces Ulmaceae, Armeniacae Semen, Panax Ginseng et Glycyrrhizae Radix, sous la forme d'une poudre ou d'un extrait obtenu au moyen d'un solvant usuel, et un ou plusieurs ingrédients choisis parmi des supports, des anti-oxydants, des agents de conservation, des agents de dissolution, des liants et des solvants, qu'on utilise habituellement dans l'industrie pharmaceutique.

3. Procédé de préparation de la composition pharmaceutique selon la revendication 2, comprenant les opérations consistant à :
(a) pulvériser Pulsatilla Radix et/ou Clematis Radix ou extraire Pulsatilla Radix et/ou Clematis Radix au moyen d'un solvant usuel utilisé dans l'industrie pharmaceutique ;
(b) pulvériser un ou plusieurs ingrédients choisis parmi l'écorce des espèces Ulmaceae, Armeniacae Semen, Panax Ginseng et Glycyrrhizae Radix, ou extraire un ou plusieurs ingrédients choisis dans le groupe comprenant l'écorce des espèces Ulmaceae, Armeniacae Semen, Panax Ginseng et Glycyrrhizae Radix ;
c) mélanger la poudre ou l'extrait de Pulsatilla Radix et/ou Clematis Radix, un ou plusieurs ingrédients choisis parmi les espèces Ulmaceae, Armeniacae Semen, Panax Ginseng et Glycyrrhizae Radix, et un ou plusieurs ingrédients choisis parmi des supports, des anti-oxydants, des agents de conservation, des agents de dissolution, des liants et des solvants, qu'on utilise habituellement dans l'industrie pharmaceutique.
